# EUROPÄISCHE PATENTSCHRIFT

(11) **EP 0 945 122 B1**
(45) Veröffentlichungstag und Bekanntmachung des Hinweises auf die Patenterteilung: **03.01.2007**
(21) Anmeldenummer: 99101736.9
(22) Anmeldetag: 11.02.1999
(51) Int. Cl.: A61K 8/19, A61K 8/365, A61K 8/63, A61K 8/67, A61Q 5/02, A61Q 5/12

(54) **Haarpflegemittel enthaltend Gamma-Oryzanol und Calciumsalzen**
Haircare composition containing gamma-oryzanol and calcium salts
Composition pour le soin des cheveux contenant du gamma-oryzanol et des sels de calcium

(30) Priorität: 11.02.1998 DE 19805428
(43) Veröffentlichungstag der Anmeldung: 29.09.1999
(73) Patentinhaber: Beiersdorf Aktiengesellschaft, 20245 Hamburg (DE)
(72) Erfinder: Riedel, Jan-Henric, Dr., 21075 Hamburg (DE); Koller, Andreas, Dr., 21035 Hamburg (DE); Argembeaux, Horst, 22525 Hamburg (DE)

(56) Entgegenhaltungen:
- DE-A- 1 617 557
- FR-A- 1 145 887
- PATENT ABSTRACTS OF JAPAN vol. 4, no. 42 (C-5) [524] & JP 55 017365 A (HOTSUKOU YUSHI)
- PATENT ABSTRACTS OF JAPAN vol. 018, no. 118 (C-1172) & JP 05 310526 A (EISAI CO.)
- "The Merck Index, Twelfth Edition" 1996, MERCK RESEARCH LABORATORIES , WHITEHOUSE STATION, NJ

## Beschreibung

Die vorliegende Erfindung betrifft Haarpflegezubereitungen oder Haarpflegemittel mit einem Gehalt an Substanzen, de die Kopfhaut und/oder das Haar, aber auch die Zubereitungen selbst vor unerwünschten Oxidationsprozessen schützen. In einer bevorzugtenAusführungsform betrifft die vorliegende Erfindung Wirkstoffkombinationen und Zubereitungen damit, die dazu dienen, das Haar und die Kopfhaut zu pflegen.

Oxidative Prozesse schädigen Stoffe unterschiedlichster Natur (z.B. Haut, Haare und Wolle, aber auch Lacke und Kunststoffe, um nur einige zu nennen) zum Teil in erheblichem Maße. Die Stoffe ändern dabei ihre physikalischen und chemischen Eigenschaften: sie "altem". Zur Verzögerung oder sogar Inhibierung der Alterung von Stoffen werden im allgemeinen sogenannte Alterungsschutzmittel verwendet Insbesondere die schädigende Wirkung des ultravioletten Teils der Sonnenstrahlung auf organische, aber auch anorganische Stoffe ist allgemein bekannt. Für den Menschen sind dabei die Schädigung von Haut und Haaren und die Verzögerung oder Verhinderung derselben durch den Einsatz von Lichtschutzmitteln von besonderer Bedeutung.

Der ganze menschliche Körper mit Ausnahme der Lippen, der Handinnenflächen und der Fußsohlen ist behaart, zum Großteil allerdings mit kaum sichtbaren Wollhärchen. Wegen der vielen Nervenenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares dürfte allerdings heute darin bestehen, das Aussehen des Menschen in charakteristischer Weise mitzugestalten. Ähnlich wie die Haut erfüllt es eine soziale Funktion, da es über sein Erscheinungsbild erheblich zu zwischenmenschlichen Beziehungen und zum Selbstwertgefühl des Individuums beiträgt.

Das Haar besteht aus dem frei aus der Haut herausragenden Haarschaft - dem keratinisierten (toten) Teil, der das eigentlich sichtbare Haar darstellt - und der in der Haut steckenden Haarwurzel - dem lebenden Teil, in dem das sichtbare Haar ständig neu gebildet wird. Der Haarschaft seinerseits ist aus drei Schichten aufgebaut: einem zentralen Teil - dem sogenannten Haarmark (Medulla), welches allerdings beim Menschen zurückgebildet ist und oft gänzlich fehlt - ferner dem Mark (Cortex) und der äußeren, bis zu zehn Lagen starken Schuppenschicht (Cuticula), die das ganze Haar umhüllt.

Das menschliche Haar ist, sofern keine krankhaften Veränderungen vorliegen, in seinem frisch nachgewachsenen Zustand praktisch nicht zu verbessern. Der in der Nähe der Kopfhaut befindliche Teil eines Haares weist dementsprechend eine nahezu geschlossene Schuppenschicht auf. Insbesondere die Schuppenschicht als Außenhülle des Haares, aber auch der innere Bereich unterhalb der Cuticula sind besonderer Beanspruchung durch Umwelteinflüsse ausgesetzt.

Wesentliche Einflüsse für den Qualitätsverlust eines Haares während seiner Alterung sind der Einfluß des Sonnenlichts, mechanische Belastungen durch intensives Kämmen oder Bürsten, aber auch Haarbehandlungen, wie Haarfärbungen und insbesondere Blondierungen sowie Haarverformungen, beispielsweise Dauerwellverfahren. Besonders oxidative Belastungen führen demnach häufig zu einer Schädigung des Haares.

Sowohl UV-A- als auch UV-B-Strahlung haben einen schädigenden Einfluß auf das Haar, der sich beispielsweise darin äußert, daß bestimmte Aminosäuren wie Cystin und Methionin abgebaut oder Schwefel-Schwefel-Bindungen des Keratins gespalten werden, was im schlimmsten Fall eine Zerstörung des Haars zur Folge haben kann. Weiterhin stellen Haar und Kopfhaut Teile des Körpers dar, die aufgrund ihrer Position beim Aufenthalt im Freien einer erheblichen Menge an UV-Strahlung ausgesetzt sind.

Bei besonders aggressiver Beanspruchung, beispielsweise der Bleichung mit Oxidantien wie Wasserstoffperoxid, bei welcher die im Cortex verteilten Pigmente oxidativ zerstört werden, kann auch das Innere des Haars in Mitleidenschaft gezogen werden. Soll menschliches Haar dauerhaft gefärbt werden, kommen in der Praxis lediglich oxidierende Haarfärbeverfahren in Betracht. Beim oxidativen Haarfärben erfolgt die Ausbildung des Farbstoffchromophoren durch Reaktion von Präkursoren (Phenole, Aminophenole, seltener auch Diamine) und Basen (meistens p-Phenylendiamin) mit dem Oxidationsmittel, zumeist Wasserstoffperoxid. Gewöhnlich werden dabei Wasserstoffperoxidkonzentrationen um 6% verwendet.

Üblicherweise wird davon ausgegangen, daß neben der Färbewirkung auch eine Bleichwirkung durch das Wasserstoffperoxid erfolgt. In oxidativ gefärbtem menschlichem Haar sind, ähnlich wie bei gebleichtem Haar, mikroskopische Löcher an den Stellen, an denen Melaningranula vorlagen, nachweisbar. Tatsache ist, daß das Oxidationsmittel Wasserstoffperoxid nicht nur mit den Farbvorstufen, sondern auch mit der Haarsubstanz reagiert und dabei unter Umständen eine Schädigung des Haares bewirken kann.

Oxidative Einflüsse, wie beispielsweise chemische Haarbehandlungen oder Sonnenlicht, setzen demnach die Festigkeit und die Elastizität des Haares herab und führen zu einer Zerstörung von Melanin. Augenscheinlich wird dies insbesondere bei dunkelhaarigen Personen, deren Haar im Sommer durch das intensive Sonnenlicht deutlich aufgehellt wird. Unter dem Bergriff "oxidativer Einfluß" ist im Sinne der vorliegenden Erfindung sowohl der Einfluß von oxidierend wirkenden Substanzen zu verstehen als auch die oxidative Wirkung von durch Strahlung, namentlich Licht, insbesondere UV-Licht, hervorgerufenen Folgeprodukten.

Ein Ziel der Haarpflege ist es, Kopfhaut und -haar vor oxidativen Einflüssen zu schützen und den Naturzustand des frisch nachgewachsenen Haares über einen möglichst langen Zeitraum zu erhalten und im Fall eines Verlusts wieder herzustellen. Seidiger Glanz, geringe Porosität und ein angenehmes, glattes Gefühl gelten als Merkmale für natürliches, gesundes Haar.

Seit Ende des vergangenen Jahrhunderts werden Produkte zur Haarpflege gezielt entwickelt. Dies führte zu einer Vielzahl von Präparaten sowohl für die allgemeine Haarpflege als auch zur Behebung von Anomalien des Haares und der Kopfhaut. Im allgemeinen werden heutzutage Haarpflegekosmetika verwendet, welche entweder dazu bestimmt sind, nach dem Einwirken aus dem Haar wieder ausgespült zu werden, oder welche auf dem Haar verbleiben sollen. Letztere können so formuliert werden, daß sie nicht nur der Pflege des einzelnen Haars dienen, sondern auch das Aussehen einer Frisur insgesamt verbessern, beispielsweise dadurch, daß sie dem Haar mehr Fülle verleihen, die Frisur über einen längeren Zeitraum fixieren oder die Frisierbarkeit verbessern.

Die Verwendung von Antioxidantien, also Substanzen, die Oxidationsprozesse verhindern, in Kosmetika ist an sich bekannt. Antioxidantien, die in der Kosmetik Verwendung finden, sind beispielsweise Tocopherole, Gallensäurederivate, Sesamol und Flavonoide. Antioxidantien werden hauptsächlich als Schutzsubstanzen gegen der Verderb der sie enthaltenden Zubereitungen verwendet.

Auch Tocopherole, insbesondere Vitamin E, sind natürlich prinzipiell geeignet, Oxidationsprozesse zu verhindern, und finden dementsprechend häufig in Kosmetika Verwendung. Allerdings haben Tocopherole den Nachteil, daß sie im allgemeinen sehr reaktiv sind und daher zum Teil bereits in der Zubereitung abreagieren. Dies führt dazu, daß nur ein kleiner Teil der Einsatzmenge den zu schützenden Körperteil überhaupt erreicht, so daß die erzielte Wirkung weit hinter der erhofften zurückbleibt.

Eine Aufgabe der vorliegenden Erfindung war, die Nachteile des Standes der Technik zu beseitigen. Insbesondere sollten kosmetische Wirkstoffe bzw. Zubereitungen, solche Wirkstoffe enthaltend, zur Verfügung gestellt werden, bei deren Verwendung die Schädigung der Kopfhaut und/oder des Haares durch oxidativen Einfluß gemindert, wenn nicht gänzlich verhindert werden kann.

Erfindungsgemäß wird diese Aufgabe gelöst und werden die Nachteile des Standes der Technik beseitigt.

Der Stand der Technik kennt zwar Nährlösungen enthaltend Calcium-Salze und Oryzanol (Deutsche Offenlegungsschrift 1617557), Auch ist dem Fachmann die antioxidative Wirkung von Oryzanol auf Cytochrom Modell-Systemen aus dem Merck index (12. Ausgabe, Seite 1181) bekannt, doch konnten diese Offenbarungen nicht den Weg zur vorliegenden Erfindung weisen.

Gegenstand der Erfindung sind Haarpflegemittel, dadurch gekennzeichnet, daß sie Gamma-Oryzanol und ein Calciumsalz oder mehrere Calciumsalze, ausgewählt aus der Gruppe, gebildet von Calciumpanthotenat, Calciumchlorid und Calciumlactat, enthalten.

Gegenstand der Erfindung ist weiterhin die Verwendung von Wirkstoffkombinationen und Haarpflegemitteln, die diese enthalten, bestehend aus Gamma-Oryzanol und einem Calciumsalz oder mehreren Calciumsalzen, ausgewählt aus der Gruppe, gebildet von Calciumpanthotenat, Calciumchlorid und Calciumlactat, zum Schutz haarkosmetischer Zubereitungen und/oder der Kopfhaut und/oder des Haares vor unerwünschten Oxidationsprozessen.

Bevorzugte haarkosmetische Zubereitungen sind Haarpflegemittel.

Die erfindungsgemäßen Wirkstoffkombinationen und Zubereitungen, diese Wirkstoffkombinationen enthaltend, mindern die Schädigung der Kopfhaut und/oder des Haares durch oxidative Einflüsse besser als Wirkstoffe, Wirkstoffkombinationen und Zubereitungen des Standes der Technik. Insbesondere pflegen sie durch oxidativen Streß geschädigtes oder strapaziertes Haar bzw. beugen solchen Schäden vor.

Gamma-Oryzanol ist in der Literatur beschrieben (CAS-Nr.: 11042-64-1 bzw. 12738-23-7). Gamma-Oryzanol ist keine einheitliche Verbindung, sondern ein Gemisch verschieden strukturierter Ferulasäureester. Gamma-Oryzanol besteht hauptsächlich aus den Estern der Ferulasäure mit den Triterpenalkoholen Cycloartenol und 24-Methylencylcloartenol. Ferner enthält es geringe Mengen an Estern mit Sterolen, namentlich mit Camesterol, Stigmasterol und β-Sitosterol.

Gamma-Oryzanol ist im Handel erhältlich, beispielsweise unter dem Handelsnamen Gamma-Oryzanol (Lieferant: Jan Dekker oder Henry Lamotte, DE).

Vorteilhaft enthalten erfindungsgemäße Zubereitungen 0,001 bis 5,0 Gew.-% Gamma-Oryzanol, bevorzugt 0,01 bis 2,0 Gew.-%, jeweils bezogen auf das Gesamtgewicht der Zubereitungen.

Vorteilhaft enthalten erfindungsgemäße Zubereitungen 0,01 bis 25 Gew.-% eines oder mehrerer Calciumsalze, bevorzugt 0,02 bis 5 Gew.-%, jeweils bezogen auf das Gesamtgewicht der Zubereitungen.

Als Calciumsalz wird Calciumlactat bevorzugt. Die Calciumsalze sind im Handel erhältlich. Das Salz der rechtsdrehenden Milchsäure wird bevorzugt.

Die Haarpflegemittel und Zubereitungen, die erfindungsgemäße Wirkstoffkombinationen enthalten, sind topische Zubereitungen. Diese können wie üblich zusammengesetzt sein und zur Behandlung und der Pflege der Kopfhaut und/oder der Haare oder als Lichtschutzpräparat dienen. Zur Anwendung werden die erfindungsgemäßen Zubereitungen in der für Kosmetika und Haarpflegemittel üblichen Weise auf die Kopfhaut und die Haare in ausreichender Menge aufgebracht.

Vorteilhaft können Zubereitungen im Sinne der vorliegenden Erfindung als Haarkur oder Haarspülung vorliegen.

Die Mittel gemäß der Erfindung können beispielsweise als aus Aerosolbehältern, Quetschflaschen oder durch eine Pump-, Sprüh- oder Schaumvorrichtung versprühbare Präparate vorliegen, jedoch auch in Form eines aus normalen Flaschen und Behältern auftragbaren Mittels.

Als Treibmittel für aus Aerosolbehältern versprühbare kosmetische oder dermatologische Zubereitungen im Sinne der vorliegenden Erfindung, sind die üblichen bekannten leichtflüchtigen, verflüssigten Treibmittel, beispielsweise Dimethylether, Kohlenwasserstoffe (Propan, Butan, Isobutan) geeignet, die allein oder in Mischung miteinander eingesetzt werden können. Auch Druckluft, Stickstoff, Stickstoffdioxid oder Kohlendioxid oder Gemische aus diesen Substanzen sind vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Vorteilhaft enthalten erfindungsgemäße Zubereitungen neben einem wirksamen Gehalt an erfindungsgemäßen Wirkstoffkombinationen ferner übliche Wirk-, Inhalts-, Zusatz- und/oder Hilfsstoffe.

Als Haarpflegemittel wird eine Vielzahl von Produkten bezeichnet, deren wichtigste Vertreter Vorbehandlungsmittel, Haarwässer und Haarkurmittel sind.

Grundstoffe für Haarpflegemittel sind z.B. Fettalkohole, Wachse, Paraffine, Vaseline, Paraffinöl und Lösemittel.

Fettalkohole sind z.B. gerad- oder verzweigtkettige aliphatische einwertige Alkohole mit 6 - 22 C-Atomen im Molekül. In der Kosmetik werden vorzugsweise geradkettige Fettalkohole mit einer Kettenlänge von 12 - 18 C-Atomen verwendet. Diese Fettalkohole sind weiche, farblose Massen, praktisch ungiftig und gut hautverträglich. Fettalkohole werden bevorzugt zur Herstellung von Haarkuren und Frisiercremes verwendet, wobei dem Cetylalkohol und dem Stearylalkohol besondere Bedeutung zukommt.

Wachse sind Fettsäureester, die in tierischen und pflanzlichen Produkten vorkommen, aber auch synthetisch hergestellt werden können. Das wohl bekannteste natürlich vorkommende Wachs ist das Bienenwachs, das als Hauptbestandteile Cerin und Myricin enthält. Wachs ist jedoch ein Oberbegriff für eine Reihe natürlich oder künstlich gewonnener Stoffe, die in der Regel halbfeste, weiße, geruchlose und in Wasser unlösliche Massen darstellen.

Paraffine im kosmetischen Sinn sind weiße, geruchlose Massen aus geradkettigen hochmolekularen Kohlenwasserstoffen. Wegen ihrer den der Wachse vergleichbaren Eigenschaften werden sie auch oft als Erdölwachse bezeichnet.

Vaseline ist ein Gemisch von verzweigtkettigen Paraffinen mit geringem Anteil an zyklischen Paraffinen. Es ist eine weiche, transparente und in Wasser unlösliche Masse mit geringem Eigengeruch, die bei der Aufbereitung des Erdöls anfällt.

Paraffinöl ist ein Gemisch gesättigter flüssiger Kohlenwasserstoffe. Es ist unlöslich in Wasser, aber mischbar mit Fettalkoholen und Wachsen. Es wird als Zusatz in Haarpflegemitteln zur Konsistenzregulierung verwendet.

Lösemittel spielen in der Kosmetik eine erhebliche Rolle. Von der großen Anzahl Lösemittel, die zur Verfügung stehen, hat Ethanol die größte Bedeutung. Es wird zur Herstellung von Haarwässern verwendet, in denen es wegen seiner desinfizierenden Eigenschaften gleichzeitig die Funktion eines Wirkstoffes erfüllt.

Hilfstoffe können verwendet werden, um bestimmte Eigenschaften der Haarpflegemittel, z.B. Konsistenz, Temperatur- und Lichtstabilität, Aussehen und Geruch, zu verbessern sowie deren Herstellung zu erleichtern. Zugesetzt werden z.B. nach Bedarf:
- Emulgatoren, um die Grenzflächenspannung zwischen zwei an sich nicht mischbaren Phasen so weit herabzusetzen, daß deren feine Vermischung möglich wird,
- Verdickungsmittel, um die Stabilität von Emulsionen zu erhöhen und deren Viskosität einzustellen,
- UV-Absorber, um die Lichtstabilität der in Haarpflegemitteln enthaltenen Farbstoffe und anderer lichtempfindlicher Komponenten zu verbessern. Daneben dienen sie dem Schutz des Haares vor Lichteinflüssen.
- Konservierungsmittel, um mikrobieller Zersetzung vorzubeugen.
- Antioxidantien, um Geruchsveränderungen, die durch Oxidationsvorgänge hervorgerufen werden können, zu verhindern.
- Farbstoffe, um Haarpflegemitteln ein ansprechendes Aussehen zu verleihen.
- Parfümöle, um Haarpflegemitteln einen angenehmen Duft zu verleihen und Nebengerüche der Rohstoffe zu überdecken.

Die Menge der Grundstoffe beträgt beispielsweise 85 bis 99,999 Gew.-%, vorzugsweise 90 bis 99,99 Gew.-%, jeweils bezogen auf das Gesamtgewicht der Zubereitung.

Quartäre Ammonium-Verbindungen sind eine wichtige Gruppe der speziellen Wirkstoffe, die zur Herstellung von Haarpflegemitteln verwendet werden. Haarpflegemittel, insbesondere Haarkuren, erhalten wesentliche Eigenschaften wie Verbesserung von Kämmbarkeit sowie Griff und Verhinderung statischer Aufladung der Haare vornehmlich durch den Einsatz quartärer Ammonium-Verbindungen.

Die Eigenschaften quartärer Ammonium-Verbindungen werden durch die kationische Gruppe einerseits und durch die Art der lipophilen Reste dieser Gruppe andererseits bestimmt. Geeignet sind z.B. die Verbindungen, in denen ein bis zwei Reste längerkettige Alkyl-Gruppen, wie Lauryl-, Cetyl- oder Stearyl-Gruppen, und die verbliebenen Reste Methyl-Gruppen sind. Produkte dieses Typs werden vorzugsweise als Chloride, Bromide und Methosulfate eingesetzt.

Geeignet sind auch polymere quartäre Ammonium-Verbindungen, Makromoleküle, deren wesentliches Merkmal das Vorhandensein mehrerer quartärer Ammonium-Gruppen im Molekül ist. Dadurch wird ihre Haftfähigkeit am Haar deutlich erhöht.

Besonders vorteilhaft zu verwendende kationische Tenside sind
1. Alkylamine
2. Alkylimidazole
3. Ethoxylierte Amine und
4. Quaternäre Tenside.
5. Esterquats

Quaternäre Tenside erhalten mindestens ein N-Atom, das mit 4 Alkyl- oder Arylgruppen kovalent verbunden ist. Dies führt, unabhängig vom pH-Wert, zu einer positiven Ladung. Vorteilhaft sind Alkylbetain, Alkylamidopropylbetain und Alkyl-amidopropylhydroxysultain. Die erfindungsgemäß verwendeten kationischen Tenside können ferner bevorzugt gewählt werden aus der Gruppe der quaternären Ammoniumverbindungen, insbesondere Benzyltrialkylammoniumchlorid, ferner Alkyltrialkylammoniumsalze, beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

Monomere oder polymere quartäre Ammonium-Verbindungen werden in Haarspülungen und Haarkuren z.B. in Konzentrationen von 0,5 - 5 Gew.-% eingesetzt. Dazu gehören Cetrimonium Chloride wie es unter der Bezeichnung Dehyquart A von der Fa. Henkel angeboten wird oder Distearoylethyl Hydroxyethylmonium Methosulfate wie es unter der Bezeichnung Dehyquart F 75 von der Fa. Henkel angeboten wird.

Liegen die kosmetischen oder dermatologischen Zubereitungen in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wird, so handelt es sich dabei z.B. um Emulsionen, die gegebenenfalls oberflächenaktive Substanzen enthalten, deren Konzentration zwischen 0,1 und 10 Gew.-%, vorzugsweise zwischen 0,2 und 5 Gew-%, liegen kann.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine Emulsion dar und enthält die erfindungsgemäßen Kombinationen. Es ist allerdings gegebenenfalls vorteilhaft, wenn die erfindungsgemäße Lotion in Form einer Mikroemulsion oder einer wäßrigen oder wäßrig-alkoholischen Lösung vorliegt.

Erfindungsgemäß können kosmetische Zubereitungen zur Behandlung und Pflege der Haare als Gele vorliegen, die organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z.B. Aluminiumsilikate wie beispielsweise Bentonite, oder ein Gemisch aus Polyethylenglykol und Polyethylenglycolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Die vorstehenden Prozentangaben beziehen sich auf das Gesamtgewicht der Zubereitungen.

Die erfindungsgemäßen Zubereitungen können kosmetische Hilfsstoffe enthalten, wie sie üblicherweise in solchen Zubereitungen verwendet werden, z.B. Konservierungsmittel, Parfüme, Substanzen zum Verhindern des Schäumens, Schaumstabilisatoren, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, rückfettende Agentien, Fette, Öle, Wachse, Alkohole, Polyole und deren toxikologisch verträglichen Ether und Ester, verzweigte und/oder unverzweigte Kohlenwasserstoffe, weitere Antioxidantien, Stabilisatoren, pH-Wert-Regulatoren, Konsistenzgeber, Bakterizide, Desodorantien, antimikrobielle Stoffe, Antistatika, UV-Absorber, Komplexierungs- und Sequestrierungsagentien, Perlglanzagentien, Polymere, Elektrolyte, organische Lösungsmittel, Silikonderivate, Pflanzenextrakte, Vitamine und/oder andere Wirkstoffe oder andere übliche Bestandteile einer kosmetischen oder dermatologischen Formulierung. Auch Lösungsvermittler, z.B. zur Einarbeitung hydrophober Komponenten wie z.B. von Parfümzubereitungen können enthalten sein.

Die Gesamtmenge der Hilfsstoffe beträgt beispielsweise 0,001 bis 15 Gew.-%, vorzugsweise 0,01 bis 10 Gew.-%, jeweils bezogen auf das Gesamtgewicht der Zubereitung.

Die Menge der Verdickungsmittel beträgt beispielsweise 0,05 bis 5,0 Gew.-%, vorzugsweise 0,1 bis 3,0 Gew.-%, insbesondere 0,15 bis 2,0 Gew.-%, jeweils bezogen auf das Gesamtgewicht der Zubereitung.

Der Wassergehalt der Zubereitungen beträgt beispielsweise 60 bis 95 Gew.-%, vorzugsweise 75 bis 95 Gew.-%, insbesondere 80 bis 90 Gew.-%, jeweils bezogen auf das Gesamtgewicht der Zubereitung.

Erfindungsgemäß können als weitere Antioxidantien alle für kosmetische und/oder dermatologische Anwendungen geeigneten oder gebräuchlichen Antioxidantien verwendet werden.

Die Gesamtmenge der Antioxidantien beträgt beispielsweise 0,001 bis 2 Gew.-%, vorzugsweise 0,01 bis 1 Gew.-%, jeweils bezogen auf das Gesamtgewicht der Zubereitung.

Vorteilhaft werden weitere Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Vitamin C und Derivate (z. B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Vorteilhaft können erfindungsgemäße Zubereitungen außerdem Substanzen enthalten, die UV-Strahlung im UV-B-Bereich absorbieren, wobei die Gesamtmenge der Filtersubstanzen z.B. 0,001 Gew.-% bis 30 Gew.-%, vorzugsweise 0,05 bis 10 Gew.-%, insbesondere 0,1 bis 1,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut, insbesondere die Kopfhaut dienen.

Enthalten die erfindungsgemäßen Emulsionen UV-B-Filtersubstanzen, können diese vorteilhaft wasserlöslich sein. Vorteilhafte wasserlösliche UV-B-Filter sind z.B.:
- Salze der 2-Phenylbenzimidazol-5-sulfonsäure wie ihr Natrium-, Kalium- oder ihr Triethanolammonium-Salz, sowie die 2-Phenylbenzimidazol-5-sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Es kann auch von Vorteil sein, erfindungsgemäße Zubereitungen mit UV-A-Filtern zu versetzen, die bisher üblicherweise in kosmetischen Zubereitungen enthalten sind. Es können die für die UV-B-Kombination verwendeten Mengen eingesetzt werden.

Das Gewichtsverhältnis der Wirkstoffe Oryzanol/Calciumsalze zueinander in den Kombinationen kann in einem breiten Bereich variiert werden und kann beispielsweise 100 : 1 bis 1 : 100, vorzugsweise 10 : 1 bis 1 : 10 und insbesondere aber auch 1:1 betragen.

Die Herstellung der erfindungsgemäßen Zubereitungen kann in der üblichen Weise durch Mischen der einzelnen Bestandteile erfolgen. Die Wirkstoffe der erfindungsgemäßen Kombinationen oder auch die vorgemischten Bestandteile der erfindungsgemäßen Kombinationen können im Mischvorgang zugegeben werden.

Der pH-Wert der Zubereitungen kann in bekannter Weise durch Zugabe von Säuren oder Basen eingestellt werden, vorzugsweise durch Zugabe von Puffergemischen, z. B. auf Basis von Citronensäure/Citrat oder Phosphorsäure Phosphat-Puffergemischen. Vorzugsweise liegt der pH-Wert unter 10, z.B. im Bereich von 2-7, insbesondere im Bereich von 3-5.

Alle Mengenangaben, Anteile und Prozentanteile sind, soweit nicht anders angegeben, auf das Gewicht und die Gesamtmenge bzw. auf das Gesamtgewicht der Zubereitungen oder der jeweiligen Mischung bezogen.

Die folgenden Beispiele verdeutlichen die Erfindung.

Als Gamma-Oryzanol wird in den Beispielen das Handelsprodukt Gamma-Oryzanol der Firma Jan Dekker, DE, verwendet.

Die Mengenangaben in den Beispielen sind Gew.-%.

### Beispiele 1-3

### Haarkur

| | **1** | **2** | **3** |
|---|---|---|---|
| Hydroxypropylmethylcellulose | 0,5 | 0,5 | 0,5 |
| Cetrimoniumbromid | 1,0 | 1,0 | 1,0 |
| Glycerin | 3,0 | 3,0 | 3,0 |
| Cetearylalkohol | 2,5 | 2,5 | 2,5 |
| Glycerylstearat | 2,0 | 2,0 | 2,0 |
| Gamma-Oryzanol | 0,02 | 0,1 | 0,5 |
| Calciumlactat | 0,5 | 0,1 | 0,02 |
| Konservierungsmittel, Parfüm, | | | |
| pH-Einstellung | q.s. | q.s. | q.s. |
| Wasser, VES (vollentsalzt) | ad 100,0 | ad 100,0 | ad 100,0 |

Der pH-Wert wird auf 3,5 eingestellt.

### Beispiele 4-6

### Haarspülung

| | **4** | **5** | **6** |
|---|---|---|---|
| Behentrimoniumchlorid | 1,0 | 1,0 | 1,0 |
| Glycerin | 3,0 | 3,0 | 3,0 |
| Hydroxyethylcellulose | 0,2 | 0,2 | 0,2 |
| Cetearylalkohol | 3,0 | 3,0 | 3,0 |
| Gamma-Oryzanol | 0,20 | 0,05 | 0,01 |
| Calciumlactat | 0,1 | 0,2 | 1,0 |
| Konservierungsmittel, Parfüm, | | | |
| pH-Einstellung | q.s. | q.s. | q.s. |
| Wasser, VES | ad 100,0 | ad 100,0 | ad 100,0 |

Der pH-Wert wird auf 3,0 eingestellt.

## Patentansprüche

1. Haarpflegemittel, **dadurch gekennzeichnet, daß** sie
a) quartäre Ammonium-Verbindungen,
b) Gamma-Oryzanol und
c) ein Calciumsalz oder mehrere Calciumsalze, ausgewählt aus der Gruppe, gebildet von Calciumpanthotenat, Calciumchlorid und Calciumlactat, enthalten.

2. Zubereitungen nach Anspruch 1, **dadurch gekennzeichnet, daß** zusätzlich weitere Tenside und/oder kosmetische oder dermatologische Hilfs-, Zusatz- und/oder Wirkstoffe enthalten sind.

3. Zubereitungen nach Anspruch 1, **dadurch gekennzeichnet, daß** der Gehalt an Oryzanol 0,001 bis 5,0 Gew.-%, bezogen auf das Gesamtgewicht der Zubereitungen, beträgt.

4. Zubereitungen nach Anspruch 1, **dadurch gekennzeichnet, daß** der Gehalt an einem oder mehreren Calciumsalzen 0,01 bis 25 Gew.-%, bevorzugt 0,02 bis 5 Gew.-%, jeweils bezogen auf das Gesamtgewicht der Zubereitungen, beträgt.

5. Zubereitungen nach einem der vorhergehenden Ansprüche, **dadurch gekennzeichnet, dass** als quartäre Ammonium-Verbindungen Benzyltrialkylammoniumchlorid, Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate eingesetzt werden.

6. Zubereitungen nach einem der vorhergehenden Ansprüche, **dadurch gekennzeichnet, dass** die quartären Ammonium-Verbindungen in Konzentrationen von 0,5-5 Gew.-% eingesetzt werden.

7. Zubereitungen nach einem der vorhergehenden Ansprüche, **dadurch gekennzeichnet, dass** die Zubereitung als Gel vorliegt, das mit einem organischen Verdickungsmittel gewählt aus der Gruppe Gummiarabikum, Xanthangummi, Natriumalginat. Cellulose-Derivate, vorzugsweise Methylcellulose, Hydroxymethylcellulose, Hydroxyethylcellulose, Hydroxypropylcellulose, Hydroxypropylmethylcellulose oder einem anorganischen Verdickungsmittel, gewählt aus der Gruppe der AluminiumsiliKate wie Bentonite, oder einem Gemisch aus Polyethylenglykol und Polyethylenglycolstearat oder-distearat, verdickt ist.

8. Zubereitungen nach einem der vorhergehenden Ansprüche, **dadurch gekennzeichnet, dass** die Zubereitung das Verdickungsmittel in einer Menge von 0,1-30 Gew.-% enthält.

9. Zubereitungen nach einem der vorhergehenden Ansprüche, **dadurch gekennzeichnet, dass** die Zubereitung einen pH-Wert von 2 bis 7 aufweiset.

## Claims

1. Haircare compositions **characterized in that** they comprise
a) quaternary ammonium compounds,
b) gamma-oryzanol and
c) one or more calcium salts chosen from the group formed by calcium panthotenate, calcium chloride and calcium lactate.

2. Preparations according to Claim 1, **characterized in that** further surfactants and/or cosmetic or dermatological auxiliaries, additives and/or active ingredients are additionally present.

3. Preparations according to Claim 1, **characterized in that** the content of oryzanol is 0.001 to 5.0% by weight, based on the total weight of the preparations.

4. Preparations according to Claim 1, **characterized in that** the content of one or more calcium salts is 0.01 to 25% by weight, preferably 0.02 to 5% by weight, in each case based on the total weight of the preparations.

5. Preparations according to one of the preceding claims, **characterized in that** the quaternary ammonium compounds used are benzyltrialkylammonium chloride, cetyltrimethylammonium chloride or bromide, alkyldimethylhydroxyethylammonium chlorides or bromides, dialkyldimethylammonium chlorides or bromides, alkylamidoethyltrimethylammonium ether sulphates.

6. Preparations according to one of the preceding claims, **characterized in that** the quaternary ammonium compounds are used in concentrations of 0.5-5% by weight.

7. Preparations according to one of the preceding claims, **characterized in that** the preparation is in the form of a gel which is thickened using an organic thickener chosen from the group gum arabic, xanthan gum, sodium alginate, cellulose derivatives, preferably methylcellulose, hydroxymethylcellulose, hydroxyethylcellulose, hydroxypropylcellulose, hydroxypropylmethylcellulose, or an inorganic thickener chosen from the group of aluminium silicates, such as bentonites, or a mixture of polyethylene glycol and polyethylene glycol stearate or distearate.

8. Preparations according to one of the preceding claims, **characterized in that** the preparation comprises the thickener in an amount of 0.1-30% by weight.

9. Preparations according to one of the preceding claims, **characterized in that** the preparation has a pH of from 2 to 7.

## Revendications

1. Agents de soin pour cheveux, **caractérisés en ce qu'**ils contiennent
a) des composés d'ammonium quaternaire,
b) du gamma-oryzanol et
c) un sel de calcium ou plusieurs sels de calcium, choisis dans le groupe formé par le pantothénate de calcium, le chlorure de calcium et le lactate de calcium.

2. Préparations selon la revendication 1, **caractérisées en ce qu'**elles contiennent en outre d'autres agents tensioactifs et/ou des adjuvants, additifs et/ou substances actives cosmétiques ou dermatologiques.

3. Préparations selon la revendication 1, **caractérisées en ce que** la teneur en oryzanol est de 0,001 à 5,0% en poids, par rapport au poids total des préparations.

4. Préparations selon la revendication 1, **caractérisées en ce que** la teneur en un ou plusieurs sels de calcium est de 0,01 à 25% en poids, de préférence de 0,02 à 5% en poids, à chaque fois par rapport au poids total des préparations.

5. Préparations selon l'une quelconque des revendications précédentes, **caractérisées en ce qu'**on utilise comme composés d'ammonium quaternaire le chlorure de benzyltrialkylammonium, le chlorure ou le bromure de cétyltriméthylammonium, les chlorures ou les bromures d'alkyldiméthylhydroxyéthylammonium, les chlorures ou bromures de dialkyldiméthylammonium, les éthersulfates d'alkylamidéthyltriméthylammonium.

6. Préparations selon l'une quelconque des revendications précédentes, **caractérisées en ce que** les composés d'ammonium quaternaire sont utilisés en des concentrations de 0,5-5% en poids.

7. Préparations selon l'une quelconque des revendications précédentes, **caractérisées en ce que** la préparation se trouve sous forme de gel qui est épaissi avec un épaississant organique choisi dans le groupe formé par la gomme arabique, la gomme de xanthane, l'alginate de sodium, les dérivés de cellulose, de préférence la méthylcellulose, l'hydroxyméthylcellulose, l'hydroxyéthylcellulose, l'hydroxypropylcellulose, l'hydroxypropylméthylcellulose ou un épaississant inorganique, choisi dans le groupe des silicates d'aluminium tels que la bentonite, ou un mélange de polyéthylèneglycol et de stéarate ou de distéarate de polyéthylèneglycol.

8. Préparations selon l'une quelconque des revendications précédentes, **caractérisées en ce que** la préparation contient l'épaississant en une quantité de 0,1-30% en poids.

9. Préparations selon l'une quelconque des revendications précédentes, **caractérisées en ce que** la préparation présente un pH de 2 à 7.
